# FASCICULE DE BREVET EUROPEEN

(11) **EP 2 451 362 B1**
(45) Date de publication et mention de la délivrance du brevet: **19.06.2019**
(21) Numéro de dépôt: 10751983.7
(22) Date de dépôt: 08.07.2010
(51) Int. Cl.: A61B 17/04

(54) **DISPOSITIF IMPLANTABLE POUR LE RAPPROCHEMENT DE STRUCTURES ANATOMIQUES NOTAMMENT DANS LE TRAITEMENT DE LA HERNIE HIATALE**
IMPLANTIERBARE VORRICHTUNG ZUR ZUSAMMENFÜGUNG ANATOMISCHER STRUKTUREN, IM BESONDEREN BEI DER BEHANDLUNG EINER HIATUSHERNIE
IMPLANTABLE DEVICE FOR BRINGING TOGETHER ANATOMICAL STRUCTURES, IN PARTICULAR IN HIATAL HERNIA TREATMENT

(30) Priorité: 09.07.2009 FR 0954758
(43) Date de publication de la demande: 16.05.2012
(73) Titulaire: Cousin Biotech, 59117 Wervicq Sud (FR)
(72) Inventeur: LEROY, Joël, F-62172 Bouvigny Boyeffles (FR); DALLEMAGNE, Bernard, F-67000 Strasbourg (FR); MARESCAUX, Jacques, F-67310 Scharrachbergheim (FR); SOLECKI, Gilles, F-59390 Lannoy (FR)
(74) Mandataire: Balesta, Pierre
(86) Numéro de dépôt international: PCT/FR2010/051448
(87) Numéro de publication internationale: WO 2011/004128

(56) Documents cités:
- EP-A1- 0 129 442
- WO-A1-2007/002071
- WO-A2-2005/016176
- WO-A2-2007/022519
- US-A1- 2002 169 359
- US-A1- 2009 093 670

## Description

La présente invention concerne un dispositif implantable destiné à réaliser le rapprochement de structures anatomiques fragiles, notamment mais non exclusivement le rapprochement des piliers du diaphragme dans le traitement de la hernie hiatale.

A l'état normal, l'orifice hiatal est traversé par le segment distal de l'oesophage accompagné des nerfs vague antérieur et postérieur. La jonction entre l'estomac et l'oesophage (cardia) est normalement située en dessous du diaphragme. Le muscle diaphragmatique sépare la cavité abdominale et la cavité thoracique. Il présente trois orifices qui permettent le passage de la veine cave, de l'aorte et de l'oesophage. L'orifice de passage de l'oesophage est dénommé orifice hiatal. Il est formé essentiellement par le pilier diaphragmatique droit, avec une contribution plus faible du pilier gauche. Le pilier droit prend son origine au niveau du ligament vertébral longitudinal antérieur qui couvre les vertèbres lombaires.

Une hernie hiatale est provoquée par une altération de l'anatomie de l'orifice hiatal. Des structures anatomiques normalement confinées dans la cavité abdominale passent alors au travers dudit orifice déformé et se retrouvent en position intra-thoracique.

Quatre types de hernie hiatale peuvent alors survenir, répertoriées selon leur gravité croissante :
- le premier type est une hernie hiatale par glissement. L'orifice hiatal est élargi et la membrane phréno-oesophagienne distendue ce qui permet l'ascension de la jonction gastro-oesophagienne au dessus du diaphragme. La plupart des hernies de ce type sont asymptomatiques. Elles peuvent cependant entraîner l'apparition d'une maladie de reflux gastro-oesophagien par la perte de la compétence du mécanisme sphinctérien oesophagien inférieur.
- le second type est une hernie para-oesophagienne. La jonction gastro-oesophagienne demeure en place, mais la partie supérieure de l'estomac (fundus) migre vers le thorax au travers d'une zone de faiblesse de la membrane phréno-oesophagienne. Ce type de hernie peut entraîner des complications mécaniques ou vasculaires au niveau du bas oesophage ou de l'estomac hernié ;
- le troisième type est une hernie mixte. Elle combine des éléments des deux premiers types. Dans certains cas, la totalité de l'estomac peut se retrouver en position sus-diaphragmatique ;
- le quatrième type est caractérisé par un élargissement important de l'orifice diaphragmatique, permettant la herniation, non seulement de l'estomac, mais également d'autres structures abdominales (colon, rate).

Le traitement d'une hernie hiatale, quel que soit le type de hernie, comporte la réduction de la hernie, c'est-à-dire le repositionnement des viscères herniés en dessous du diaphragme, et la plastie de l'orifice hiatal élargi.

La plastie de l'orifice hiatal consiste en la recalibration de l'orifice hiatal autour de l'oesophage, sans comprimer mécaniquement l'oesophage tout en assurant une fermeture satisfaisante qui doit empêcher une récidive de la hernie. Cette réparation est appliquée sur une structure musculaire (piliers du diaphragme) qui n'a pas la résistance d'une structure aponévrotique et qui est sollicitée par des variations de pressions intra-abdominales (positives) et thoraciques (négatives).

La localisation de l'orifice hiatal (postérieure) accroît la difficulté du geste technique.

US 2009/093670 A1 divulgue un dispositif médical permettant de réduire la distance entre deux points du muscle cardiaque.

Ce dispositif comprend un élément longiligne, une première pièce d'appui disposée sur la longueur dudit élément qui peut être rigide puisque éventuellement dans un métal tel que l'acier inoxydable, une seconde pièce d'appui, un plug et des pièces de blocage.

Le plug peut comprendre des composants cellulaires ou chimiques qui facilitent la fibrose.

Une première technique de plastie des hernies hiatales consiste à suturer avec des fils de suture non résorbables les piliers du diaphragme, en avant et/ou en arrière de l'oesophage. Des languettes, généralement en PTFE, peuvent être intercalées entre le fil de suture et les piliers du diaphragme pour éviter de cisailler les structures musculaires, cette technique de suture est alors appelée suture appuyée. Malgré cette précaution, on observe un effet de cisaillement des piliers gauche et droit, particulièrement avec la technique de suture simple. Cette première technique réalisée par laparoscopie rapporte des taux de récidive jusqu'à 42 % supérieurs aux taux de récidive observés dans la chirurgie conventionnelle, c'est-à-dire par laparotomie. Il n'existe pas d'étude randomisée comparant la suture simple et la suture appuyée. La différence des taux de récidive entre la chirurgie ouverte et la chirurgie laparoscopique laisse suspecter l'existence de difficultés techniques propres à la chirurgie laparoscopique.

Une seconde technique consiste à renforcer la suture des piliers en la recouvrant d'une prothèse qui entoure partiellement ou totalement l'oesophage. Pour être efficace, la prothèse est invariablement proche de la paroi de l'oesophage. Une étude réalisée sur une durée courte -6 mois- démontre un taux de récidive de 9% après renforcement au moyen d'une prothèse contre 24% pour la suture simple. Ces résultats favorables doivent être contre-balancés par la description de complications sévères liées à l'utilisation du matériel prothétique. Un contact direct entre la prothèse et la paroi de l'oesophage peut provoquer soit une réaction inflammatoire fibreuse qui entraîne une sténose de l'oesophage, soit une érosion progressive de la paroi oesophagienne avec pénétration de la prothèse dans la lumière de l'oesophage. Ces complications nécessitent une reprise chirurgicale pouvant aller jusqu'à la résection de l'oesophage. La probabilité d'observer ces complications est majorée par le fait que le contact prothèse-oesophage est dynamique, entre les mouvements respiratoires du diaphragme et les mouvements péristaltiques de l'oesophage, non synchronisés.

Une troisième technique consiste à disposer une prothèse sur l'orifice hiatal sans tension, c'est-à-dire sans suture préalable des piliers. Aucun suivi à long terme n'a été rapporté à ce jour sur cette dernière technique.

FR 2.889.441 A1 décrit une prothèse de renfort utilisée pour la mise en oeuvre des seconde et troisième techniques. Cette prothèse a une forme de collerette au centre de laquelle est disposé l'oesophage.

De nombreuses études ont démontré que la récidive de la hernie hiatale est la cause principale d'échec du traitement chirurgical du reflux gastro-oesophagien et de la hernie hiatale. Elle est à l'origine de la majorité des reprises chirurgicales. La récidive est provoquée par la rupture, la distension ou une malfaçon de la plastie crurale.

Trois facteurs doivent être pris en considération lors du traitement d'une hernie hiatale :
- la longueur de l'oesophage. Elle doit être suffisante pour permettre le repositionnement sans tension de la jonction gastro-oesophagienne sous le diaphragme ;
- la qualité des piliers du diaphragme. En cas de hernie volumineuse, les piliers peuvent être très écartés et réduits à deux fines bandes musculaires fragiles ;
- la qualité de la plastie de l'orifice hiatal. 90 à 95% des hernies observées dans la maladie de reflux gastro-oesophagien sont des hernies du premier type décrit ci-dessus et donc de petite taille, avec conservation d'une musculature satisfaisante. La situation anatomique de l'orifice hiatal peut rendre difficile un geste de suture laparoscopique dont la qualité dépend significativement de l'expérience de l'opérateur.

La technique qui s'applique dans le cadre de la présente divulgation, s'agissant du traitement de la hernie hiatale, s'apparente à la première technique de plastie précitée en ce qu'elle cherche à rapprocher les piliers du diaphragme en avant et/ou en arrière de l'oesophage. Selon la présente divulgation, il ne s'agit pas de suturer les piliers du diaphragme avec des fils de suture classiques mais de mettre en oeuvre un dispositif particulièrement adapté au rapprochement desdits piliers.

Un tel dispositif n'est pas limité au traitement de la hernie hiatale mais peut s'appliquer au rapprochement de structures anatomiques fragiles, que ce soit des structures musculaires telles que la paroi abdominale, les piliers du diaphragme, le coeur, l'estomac ou l'utérus ou des structures parenchymateuses telles que les reins, le foie et les poumons.

Ledit dispositif comprend un élément longiligne, destiné à traverser de part en part les structures anatomiques à rapprocher, ledit élément longiligne comportant sur sa longueur une première pièce d'appui qui comporte une face de contact destinée à s'appliquer sur l'une des structures anatomiques à rapprocher et au moins une pièce de blocage qui est située à une distance donnée D de la face de contact de ladite première pièce d'appui. Le dispositif comprend également une seconde pièce d'appui qui comporte une face de contact destinée à s'appliquer sur une autre des structures anatomiques à rapprocher, ladite seconde pièce d'appui étant apte à coulisser sur la longueur de l'élément longiligne et étant conformée en sorte que, lors de l'implantation, la pièce de blocage est forcée à travers la seconde pièce d'appui et vient en butée arrière contre celle-ci, les faces de contact des première et seconde pièces d'appui étant tournées l'une vers l'autre et délimitant un espace apte à recevoir et enserrer les structures anatomiques à rapprocher. Enfin, chaque face de contact est, au moins en partie, apte à développer une fibrose cicatricielle.

Ainsi, selon les dispositions particulières de la présente divulgation, lorsque les structures anatomiques sont rapprochées, étant enserrées entre les faces de contact des deux pièces d'appui, et que la fibrose cicatricielle se développe dans lesdites faces, on obtient un blocage définitif en position de l'ensemble constitué par les deux pièces d'appui et l'élément longiligne de sorte que ledit élément longiligne n'a plus la possibilité de se déplacer perpendiculairement aux structures anatomiques et qu'il n'y a donc plus de risques de cisaillement desdites structures.

Etant donné qu'il s'agit de rapprocher des structures anatomiques fragiles, chaque face de contact de la pièce d'appui doit présenter, selon l'interprétation qu'il convient de faire de ce terme selon la présente divulgation, une surface suffisante pour éviter les risques de déformation voire de pénétration de la pièce d'appui dans la structure anatomique contre laquelle elle vient s'appliquer lors de l'implantation.

Ainsi la terminaison en T du dispositif décrit dans le document WO 2005/016176A ne peut en aucun cas être considéré comme une pièce d'appui apte à permettre le rapprochement de structures anatomiques fragiles, y compris lorsqu'elle vient en coopération avec l'élément de type bouton qui est illustré aux figures 34,35 de ce document.

A titre indicatif, chaque face de contact a une surface qui fait au moins 40 mm². Il peut s'agir notamment d'une face de configuration elliptique ou circulaire, dans ce dernier cas ayant un diamètre de l'ordre de 8 mm.

Contrairement aux techniques de sutures simples et appuyées encerclant en partie au moins deux structures anatomiques, telles que les piliers gauche et droit, l'élément longiligne traverse lesdites structures anatomiques, lesquelles sont maintenues entre les faces de contact des première et seconde pièces d'appui. Il n'y a donc pas de fil de suture venant directement en contact avec la surface extérieure desdites structures anatomiques, ce qui élimine les risques de cisaillement de ces dernières qui sont des structures fragiles, particulièrement en ce qui concerne les piliers du diaphragme qui sont des muscles « tendres ». Lesdites structures anatomiques, par exemple les piliers, sont enserrées entre les pièces d'appui bloquées et distantes d'une distance déterminée, qui est fonction de la distance entre la face de contact de la première pièce d'appui et la pièce de blocage assurant le blocage de la seconde pièce d'appui, de sorte que le praticien connaît précisément la distance selon laquelle il a rapproché lesdites structures anatomiques, ce qui augmente ainsi la précision de la technique opératoire.

Dans le cas du traitement de la hernie hiatale, le dispositif une fois implanté ne comporte pas de bords extérieurs susceptibles d'entrer en contact avec la paroi oesophagienne. Le dispositif est facile à mettre en place contrairement aux prothèses et sutures utilisés dans l'état de la technique ce qui diminue les risques post-opératoires de récidive. De plus, ledit dispositif étant peu encombrant peut être implanté facilement par laparoscopie. Les piliers gauche et droit viennent en appui contre les faces de contact des première et seconde pièces d'appui et sont retenus définitivement dans leur position resserrée entre ces dernières tout particulièrement après le développement de la fibrose cicatricielle. Il est bien sûr possible d'implanter le dispositif selon la présente divulgation par laparotomie.

La première pièce d'appui et la ou les pièces de blocage sont fixées ou fixables sur la longueur dudit élément longiligne par n'importe quels moyens mécaniques ou chimiques connus de l'état de la technique : clipsage, collage, soudures ultrasons ou haute fréquences..., ou encore en réalisant un noeud sur lui-même dudit élément longiligne faisant office de pièce de blocage ou de butée arrière pour ladite première pièce d'appui.

Chaque première pièce d'appui est constituée d'un corps principal rigide ou semi-rigide, et d'une pièce souple qui se projette radialement selon tout ou partie du pourtour du corps principal et qui est apte à passer d'une position d'introduction dans laquelle elle est repliée sur elle-même à une position d'implantation dans laquelle est déployée pour venir s'appliquer sur l'une des structures anatomiques à rapprocher. La face de la pièce souple qui, lors de l'implantation, est destinée à être appliquée sur la structure anatomique fait bien sûr office de face de contact selon la présente invention, étant notamment apte à développer la fibrose cicatricielle.

Ainsi, c'est la face du corps principal qui est en regard de la structure anatomique qui est à prendre en considération en ce qui concerne le serrage desdites structures anatomiques lors du rapprochement de celles-ci. Par contre, lorsque la fibrose cicatricielle s'est développée, c'est l'ensemble de la surface incluant l'extension radiale de la pièce souple au-delà du corps principal qui est à prendre en considération dans le blocage à long terme des deux structures anatomiques en position resserrée. Cette variante de réalisation est particulièrement adaptée pour les implantations qui interviennent par voie laparoscopique, puisque dans ce cas on peut utiliser pour l'introduction du dispositif un trocart de faible diamètre, correspondant sensiblement à celui du corps principal des deux pièces d'appui.

De préférence, la pièce souple, au moins dans son extension radiale au-delà du corps principal, comporte sur sa face opposée à la face de contact des moyens aptes à empêcher le développement de la fibrose cicatricielle. Il peut notamment s'agir d'une enduction ou d'une imprégnation de silicone. Le but visé par cette disposition particulière est d'éviter qu'il puisse y avoir un développement anarchique de fibrose dû à la présence de cette pièce souple s'il arrivait que celle-ci vienne en contact avec d'autres structures anatomiques que celles dont le rapprochement est recherché.

A titre d'exemple non exhaustif, le corps principal étant une pièce de configuration circulaire faisant de l'ordre de 8 mm de diamètre, l'extension radiale au-delà du corps principal présente un diamètre de 20mm.

A titre indicatif, chaque face de contact a une surface qui fait au moins 40 mm². Il peut s'agir notamment d'une face de configuration elliptique ou circulaire, dans ce dernier cas ayant un diamètre de l'ordre de 8 mm.

De préférence, chaque face de contact présente une surface qui reste supérieure à 40 mm² pour éviter les risques de transpercement de la structure anatomique, et inférieure à 1300 mm², pour éviter de générer des érosions entre la surface et la structure anatomique.

Selon une autre variante de réalisation, la face de contact de chaque pièce d'appui est constituée d'un matériau textile apte à permettre le développement d'une fibrose cicatricielle, notamment un non-tissé, par exemple de préférence en polyéthylènetéréphtalate (PET). Ce matériau textile peut occuper toute la surface de la pièce d'appui. Dans la variante particulière visée ci-dessus, il peut soit occuper uniquement la surface de l'extension radiale venant au-delà du corps principal soit occuper toute la surface de la face de contact, incluant le corps principal et l'extension radiale.

Dans une variante de réalisation, la pièce d'appui comporte un corps principal et une pièce souple qui sont, avant implantation, indépendantes l'une de l'autre, ladite pièce souple étant apte à coulisser le long de l'élément longiligne pour venir contre le corps principal et former ainsi la face de contact de la pièce d'appui.

Dans une variante, la face de contact des première et seconde pièces d'appui est sensiblement plane, de préférence elle a une forme générale d'ellipse ou de cercle.

Les faces de contact planes des première et seconde pièces d'appui sont plaquées contre les structures anatomiques, de sorte qu'elles ne peuvent cisailler ni endommager la structure musculaire fibreuse de ces dernières lorsqu'il s'agit de muscles, en particulier des piliers du diaphragme, pendant la période qui précède la fibrose cicatricielle.

De préférence, la première pièce d'appui a une forme générale sensiblement plane.

Dans une variante, le dispositif comprend un second élément longiligne fixé sur la première pièce d'appui, et au moins une pièce de blocage située sur le second élément à une distance donnée D de la face de contact de la première pièce d'appui, la seconde pièce d'appui étant apte à coulisser sur la longueur dudit second élément longiligne en sorte que, lors de l'implantation, ladite pièce de blocage est forcée à travers ladite seconde pièce d'appui et vient en butée contre celle-ci.

La seconde pièce d'appui est apte à coulisser à la fois sur le premier élément longiligne et sur le second élément longiligne.

L'emploi d'un second élément longiligne permet de renforcer la pression exercée de part et d'autre desdites structures anatomiques respectivement par les première et seconde pièces d'appui.

Le second élément traverse également lesdites structures anatomiques de sorte qu'il ne peut cisailler leurs surfaces extérieures.

Lorsque les structures anatomiques sont les deux piliers du diaphragme, le choix du nombre d'élément longiligne est fonction du renfort à apporter à la plastie du hiatus oesophagien et du type de hernie hiatale.

Dans une variante, le premier, et éventuellement le second, élément longiligne comporte plusieurs pièces de blocage successives, de préférence ayant une hauteur du même ordre, à des distances prédéterminées D de la face de contact de ladite première pièce d'appui, de préférence la distance D est comprise dans l'intervalle [10 ; 25] mm.

Le premier, et éventuellement le second, élément longiligne comporte de préférence quatre pièces de blocage, la première étant à une distance D de préférence de l'ordre de 10 mm de la face de contact de la première pièce d'appui, les pièces de blocage suivantes étant espacées les unes des autres de préférence de l'ordre de 5 mm à compter de ladite première pièce de blocage. Les pièces de blocage sont autant de repère pour le praticien pour bloquer la seconde pièce d'appui et déterminer précisément la distance selon laquelle les structures anatomiques sont rapprochées ce qui n'était pas possible avec les sutures et orthèses mis en oeuvre dans l'état de la technique, particulièrement pour le traitement de la hernie hiatale.

Les pièces de blocage disposées sur les premier et second éléments longilignes sont de préférence à égales distances de ladite première pièce d'appui de sorte que le praticien force deux pièces de blocage à la fois à travers la seconde pièce d'appui en sorte de former un espace délimité entre les première et seconde pièces d'appui ayant au moins une dimension sensiblement constante.

La hauteur d'une pièce de blocage correspond à la longueur d'une portion de premier, ou de second, élément longiligne selon laquelle ladite pièce est fixée. De préférence, les pièces de blocage sont identiques et présentent une hauteur du même ordre.

La seconde pièce d'appui présente un premier orifice permettant le passage dudit premier élément longiligne, et éventuellement un second orifice permettant le passage dudit second élément longiligne.

Le premier, et éventuellement le second, orifice permet à la seconde pièce d'appui de coulisser librement sur la longueur dudit premier, et éventuellement dudit second, élément longiligne.

Dans une variante, la seconde pièce d'appui comporte une première pièce tronconique creuse, et éventuellement une seconde pièce tronconique creuse, ayant des découpes formant des ailettes latérales et ayant un évidement central qui prolonge l'orifice de passage. Le diamètre de sortie de l'évidement de ladite pièce tronconique est inférieur à l'encombrement de la pièce de blocage, en sorte que le passage en force de la pièce de blocage à travers la seconde pièce d'appui est obtenu grâce à la déformation radiale des ailettes.

La première pièce tronconique creuse, et éventuellement la seconde pièce tronconique creuse, s'étend de préférence de la face extérieure de ladite seconde pièce d'appui de sorte que celle-ci conserve une face de contact sensiblement plane pour ne pas cisailler les structures anatomiques.

Dans une variante, la première, et éventuellement la seconde, pièce tronconique creuse comporte un épaulement interne dans sa portion d'extrémité formant un logement apte à recevoir tout ou partie de la hauteur d'une pièce de blocage.

Selon la profondeur de l'épaulement interne, tout ou partie de la hauteur de la pièce de blocage est logée à l'intérieur de la première, ou de la seconde, pièce tronconique et ce dans l'évidement central.

Les découpes latérales sont ainsi en partie, ou totalement selon la profondeur de l'épaulement, obturées par la pièce de blocage en sorte que l'élément longiligne ne puisse passer à travers lesdites ailettes et cisailler des structures anatomiques environnantes.

Dans une variante, ladite première, et éventuellement ladite seconde, pièce tronconique, est pourvue d'un protecteur, de préférence consistant dans une bande, éventuellement dans un matériau élastique, de préférence à base de silicone, ayant une largeur de l'ordre de la hauteur de ladite pièce tronconique, éventuellement majorée de tout ou partie de la hauteur de l'une des pièces de blocage, et apte à être disposée en fonctionnement sur ladite pièce tronconique et éventuellement ladite pièce de blocage, en sorte de recouvrir au moins lesdites découpes latérales.

Lorsque le dispositif comprend deux éléments longilignes, la seconde pièce d'appui comprend de préférence deux pièces tronconiques telles que décrites ci-dessus et deux desdits protecteurs, chacun étant apte à être disposé en fonctionnement sur les pièces tronconiques.

De préférence, la largeur du protecteur est déterminée en sorte qu'il recouvre à la fois les découpes latérales et tout ou partie de la hauteur de la pièce de blocage sélectionnée. Lorsque la première, et éventuellement la seconde, pièce tronconique comporte un épaulement interne, la largeur du protecteur correspond à la hauteur de ladite pièce tronconique majorée de la hauteur de la pièce de blocage moins la profondeur dudit épaulement. Cette disposition permet que la pièce de blocage, en particulier lorsqu'elle comporte une extrémité amincie voire pointue, soit recouverte par ledit protecteur pour éviter de perforer des structures anatomiques voisines.

Ce protecteur évite que le premier, éventuellement le second, élément longiligne, et éventuellement la pièce de blocage, ne passent à travers l'une des découpes latérales lors du passage en force de ladite pièce de blocage occasionnant la déformation desdites ailettes ou une fois le dispositif implanté, et ne viennent en contact avec la surface extérieure des structures anatomiques. On élimine ainsi le risque de cisaillement et la gêne que cela pourrait provoquer pour le praticien lors de la manipulation du dispositif.

La pièce de blocage, en particulier lorsqu'elle comporte une extrémité amincie voire pointue, est de préférence recouverte par ledit protecteur pour éviter de perforer des structures anatomiques voisines.

S'agissant des piliers du diaphragme qui sont des structures anatomiques très tendres et fragiles, en particulier parce que les fibres des muscles sont toutes orientées dans la direction longitudinale des piliers, compte tenu de la zone d'implantation soumise aux mouvements du diaphragme (environ 2500 pulsations du diaphragme par jour), la demanderesse a observé que, sans protecteur, si le premier élément longiligne et éventuellement la pièce de blocage passent à travers une découpe latérale et viennent en contact avec le pilier recevant la seconde pièce d'appui, ce pilier est cisaillé transversalement par ces derniers et le rapprochement des piliers opéré grâce au dispositif est alors nul. Le protecteur élimine ainsi tout risque de cisaillement des piliers du diaphragme et permet que le rapprochement des piliers opéré par le dispositif selon l'invention soit parfaitement stable.

Dans une variante, le premier, et éventuellement le second, élément longiligne est un monofilament, une tresse ou un câble rigide de faible diamètre, de préférence de l'ordre de 1,7 mm.

Dans une variante, le premier, et éventuellement le second, élément longiligne présente, à l'opposé de ladite première pièce d'appui, une extrémité rigide et courbe permettant son introduction dans lesdites structures anatomiques.

La rigidité doit être suffisante pour que ladite extrémité puisse traverser lesdites structures anatomiques et notamment les piliers. Ladite extrémité peut être formée à partir du premier ou du second élément longiligne lui-même par enduction ou imprégnation d'un polymère implantable puis mise en forme, ou par fusion du ou des polymères formant le dit premier ou second élément à l'aide d'un solvant de ces derniers. Ladite extrémité peut être également formée en rapportant sur l'extrémité libre une pièce courbe et rigide implantable moulée.

Dans une variante, la ou les pièces de blocage ont une forme générale tronconique.

Dans une variante, les première et seconde pièces d'appui et la ou les pièces de blocage sont moulées, de préférence dans un polymère choisi parmi les polymères suivants : PEEK (polyétheréthercetone), POM (polyoxyméthylène), PET (polyéthylènetéréphtalate), PP (polypropylène), PE (polyéthylène).

L'invention concerne selon un deuxième aspect un ensemble pour le rapprochement de structures anatomiques fragiles, notamment de structures musculaires telles que la paroi abdominale, les piliers du diaphragme dans le traitement de la hernie hiatale, le coeur, l'estomac ou l'utérus ou de structures parenchymateuses telles que les reins, le foie et les poumons comportant un dispositif implantable selon l'une des variantes de réalisation décrites ci-dessus et un kit d'implantation comprenant notamment un organe de traction et un poussoir.

L'invention concerne selon un troisième aspect, l'utilisation d'un ou plusieurs dispositifs implantables selon l'une des variantes de réalisation décrites ci-dessus, pour le rapprochement de structures musculaires telles que la paroi abdominale, le diaphragme, le coeur, l'estomac ou l'utérus.

L'invention concerne selon un quatrième aspect, l'utilisation d'un ou plusieurs dispositifs implantables selon l'une des variantes de réalisation décrites ci-dessus, pour le rapprochement de structures parenchymateuses telles que les reins, le foie et les poumons.

L'invention sera mieux comprise, grâce à la description ci-après, qui se rapporte à deux modes de réalisation préférés, donné à titre d'exemples non limitatifs, et expliqués avec références aux dessins schématiques annexés, dans lesquels :
- la figure 1A est une représentation schématique en perspective et vue de côté d'un premier exemple de dispositif ;
- la figure 1B est une représentation schématique en perspective et vue de côté de la seconde pièce d'appui représentée à la figure 1A ;
- la figure 1C est une représentation schématique vue selon la face intérieure de la seconde pièce d'appui représentée à la figure 1B ;
- la figure 1D est une représentation schématique vue de coté du protecteur de la pièce tronconique de la seconde pièce d'appui représentée aux figures 1B et 1C ;
- la figure 2 est une représentation schématique d'une hernie hiatale du second type ;
- les figures 3 à 8 sont des représentations schématiques des différentes étapes pour l'implantation du dispositif décrit aux figures 1A, 1B et 2 ;
- les figures 9 et 10 sont des représentations schématiques en perspective d'une variante de la pièce tronconique représentée aux figures 1B-1D ;
- les figures 11 et 12 sont des représentations schématiques vues de côté d'un second mode de réalisation du dispositif ;
- la figure 13 est une représentation schématique en perspective d'un variante du protecteur représenté à la figure 10 ;
- les figures 14 et 15 sont des représentations schématiques en coupe longitudinale de deux exemples de première pièce d'appui, selon la présente invention, dont la face de contact est une pièce souple à extension radiale.

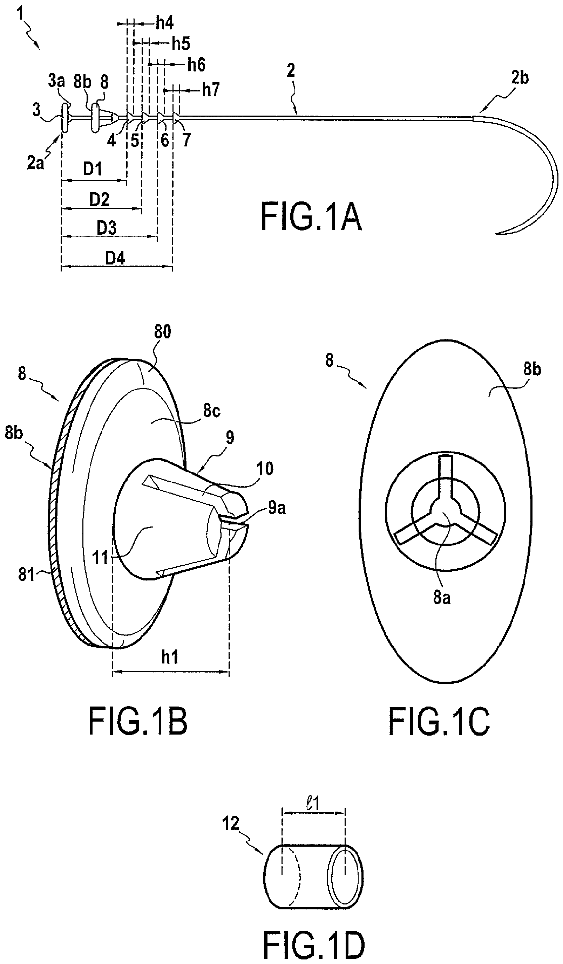

Le dispositif implantable 1 représenté à la figure 1A est destiné au traitement de la hernie hiatale. Il comprend un élément longiligne de rapprochement 2, lequel comprend sur sa longueur une première pièce d'appui 3, fixe et terminant l'une des extrémités 2a dudit élément longiligne 2, et quatre pièces de blocage 4,5,6,7 successives. Les première 4, seconde 5, troisième 6 et quatrième 7 pièces de blocage sont respectivement à des distances D1,D2,D3,D4 de la face de contact 3a de ladite première pièce d'appui 3, c'est-à-dire celle qui est destinée à venir s'appliquer contre l'une des structures anatomiques à rapprocher. Dans cet exemple particulier, D1,D2,D3,D4 valent respectivement 10 mm, 15 mm, 20 mm et 25 mm. Les pièces de blocage 4,5,6,7 ont respectivement une hauteur h4,h5,h6,h7 du même ordre, dans cet exemple précis de l'ordre de 5 mm. Les pièces de blocage 4,5,6,7 ont une forme générale tronconique dont la plus petite base est orientée sur ledit élément longiligne 2 dans une direction opposée à la première pièce d'appui 3. La première pièce d'appui 3 et les pièces de blocage 4,5,6,7 sont fixées sur la longueur dudit élément longiligne 2 par n'importe quels moyens connus de l'état de la technique, par exemple par collage.

Le dispositif 1 comprend une seconde pièce d'appui 8 ayant un orifice 8a permettant le passage dudit élément longiligne 2 et une face de contact 8b destinée à venir s'appliquer contre l'autre structure anatomique à rapprocher. Les faces de contact 3a et 8b des première 3 et seconde 8 pièces d'appui sont sensiblement planes et ont une forme générale d'ellipse ou circulaire. Elles sont toutes deux aptes à développer une fibrose cicatricielle. Dans l'exemple de réalisation illustré à la figure 1B, la seconde pièce d'appui est constituée d'un corps principal 80 et d'une pièce textile 81, qui est plaquée sur la face intérieure du corps principal 80 et qui fait office de face de contact 8b. Ainsi, dans cet exemple, c'est toute la surface du corps principal 80 qui est recouverte de la pièce textile apte à permettre le développement de la fibrose cicatricielle. Ce qui vient d'être dit en ce qui concerne la seconde pièce d'appui 8 s'applique, dans les mêmes conditions, à la première pièce d'appui 3 formée également d'un corps principal et d'une pièce textile plaquée sur la face intérieure dudit corps principal et formant la face de contact 3a de ladite première pièce d'appui 3.

La pièce textile 81 est par exemple un non-tissé, de préférence en polyéthylènetéréphtalate, ladite pièce faisant de 30 à 500 g/m² et ayant une épaisseur de l'ordre de 0.05 à 0.70 mm. Une telle pièce textile 80 permet le développement d'une fibrose lors du contact avec la structure anatomique. Cette fibrose se développe dans l'épaisseur de ladite pièce textile, ce qui permet un ancrage de ladite pièce, et donc de la pièce d'appui 8 qui lui est solidaire, sur ladite structure anatomique.

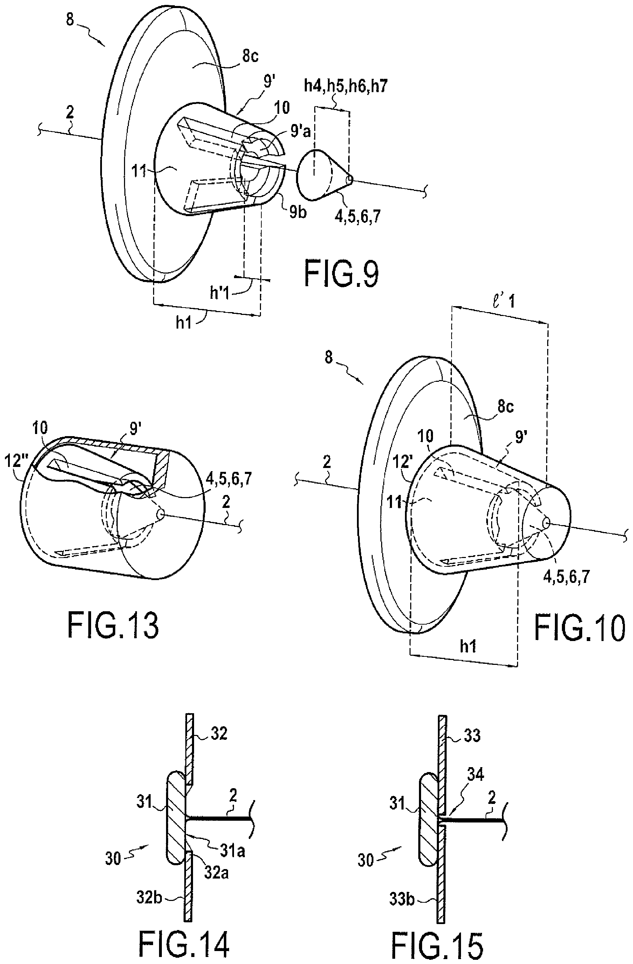

Dans les deux exemples, selon la présente invention, qui sont illustrés aux figures 14 et 15 la première pièce d'appui 30 est constituée d'un corps principal rigide ou semi-rigide 31 et d'une pièce souple qui se projette radialement selon le pourtour du corps principal 31, ladite pièce souple étant apte à passer d'une position d'introduction dans laquelle elle est repliée sur elle-même à une position d'implantation dans laquelle elle est déployée pour venir s'appliquer sur l'une des structures anatomiques à rapprocher.

Dans l'exemple illustré à la figure 14, la pièce souple 32 a une configuration annulaire n'étant solidarisée à la face intérieure 31a du corps principal 31, lui-même de configuration circulaire, que par son bord central 32a. Dans cet exemple, la face de contact de la pièce d'appui 30 est constituée par la pièce souple 32 et par la portion de surface intérieure 31a du corps principal 31, qui n'est pas recouverte par la pièce souple 32. Dans ce cas, le développement de la fibrose cicatricielle n'intervient que sur une partie de la face de contact, à savoir celle qui correspond à la pièce souple 32.

Dans l'exemple illustré à la figure 15, la pièce souple 33 est de configuration circulaire, avec simplement un trou de passage 34 pour l'élément longiligne. Dans ce cas, la face de contact 33, apte au développement de la fibrose cicatricielle, est formée dans son intégralité par la pièce souple 33.

Dans ces deux exemples, la pièce souple qui peut être un non-tissé comme dans le mode de réalisation précité, est de préférence solidarisé au corps principal 30 par toute technique connue, notamment par soudure, ultrasons ou par collage. Cependant, s'agissant de l'exemple de la figure 15, il est envisageable que la pièce souple 33 se présente comme une pièce qui, au départ, est indépendante du corps principal 31, étant montée à coulissement le long de l'élément longiligne 2 par le praticien lors de l'implantation. La solidarisation de la pièce souple 33 au corps principal 31 se fait, de façon naturelle, par l'effet de compression obtenu lors du rapprochement des structures anatomiques suivies du blocage en position resserrée des deux pièces d'appui grâce à l'action de la pièce de blocage.

Ce qui vient d'être dit, concernant les exemples des figures 14 et 15, pour la première pièce d'appui 30 s'applique bien entendu à la seconde pièce d'appui 8.

La première pièce d'appui 3 a la forme générale d'une plaque plane. La seconde pièce d'appui 8 comporte une pièce tronconique creuse 9 s'étendant de sa face extérieure 8c. La pièce tronconique 9 comporte des découpes 10 formant des ailettes latérales 11 et ayant un évidement central 9a qui prolonge l'orifice de passage 8a. Le diamètre de sortie de l'évidement 9a de ladite pièce tronconique 9 est inférieur à l'encombrement des pièces de blocage 4,5,6,7. L'élément longiligne 2 présente une extrémité rigide et courbe 2b à l'opposé de ladite première pièce d'appui 3. Le dispositif 1 comprend également un protecteur se présentant sous la forme d'une bande 12 dans un matériau élastique et implantable, de préférence à base de silicone, ayant une largeur ℓ1 de l'ordre de la hauteur h1 de ladite pièce tronconique 9, de préférence de l'ordre de la hauteur h1 ajoutée à la hauteur h4,h5,h6,h7 de l'une des pièces de blocage 4,5,6,7. Ladite bande 12 est disposée en fonctionnement sur la pièce tronconique 9 en sorte de recouvrir non seulement les découpes latérales 10, mais également la pièce de blocage 4,5,6,7 afin de protéger les structures anatomiques proches d'éventuelles perforations dues à l'extrémité amincie, globalement en forme de pointe, de la pièce de blocage 4,5,6,7.

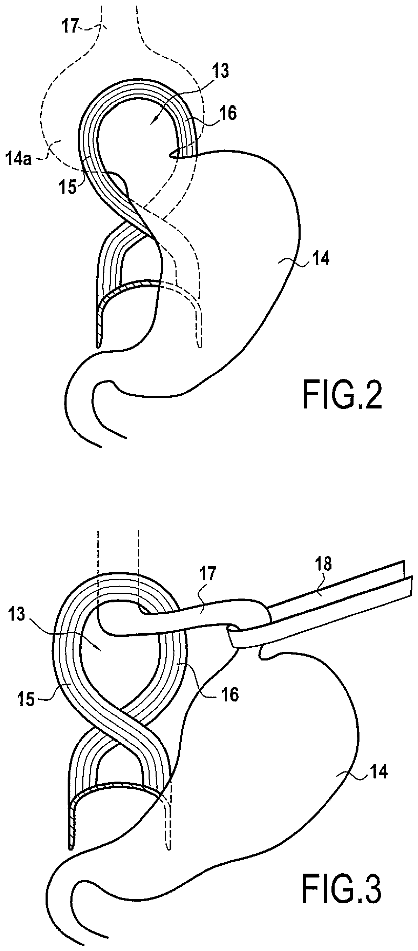

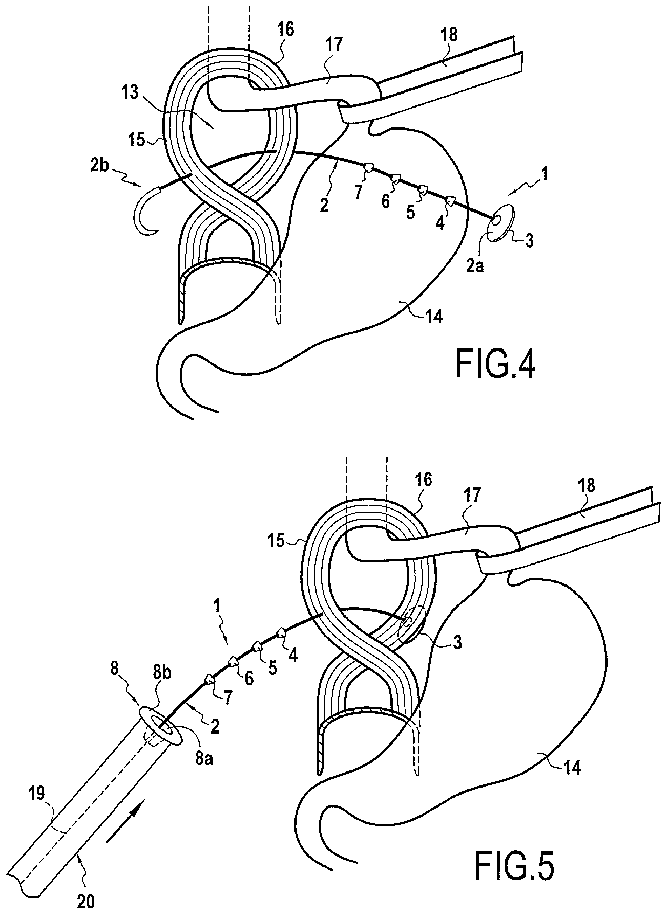

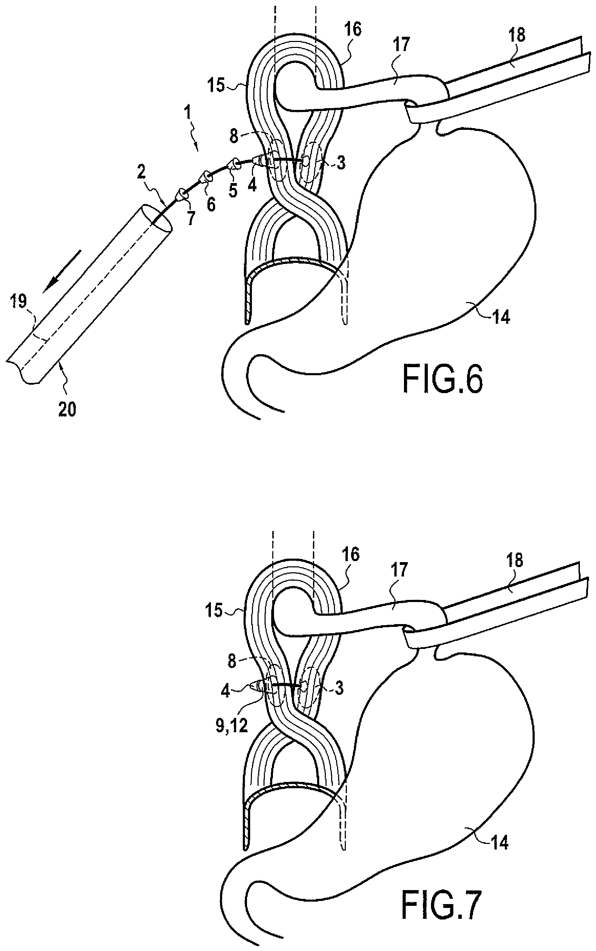

La figure 2 est une représentation schématique d'une hernie hiatale 13 mettant en évidence la remontée par le hiatus oesophagien 13 d'une partie 14a de l'estomac 14 suite à une distension entre les piliers 15,16 du diaphragme. Le dispositif 1 est implanté de préférence par laparoscopie. En premier, la base de l'oesophage 17 est écartée au moyen d'un lac 18 maintenu sous tension et se présentant sous la forme d'un cordon pour dégager les piliers 15,16 (figure 3). Puis l'élément longiligne 2 est introduit à travers les piliers 15,16, sensiblement en leurs centres, à partir de son extrémité courbe 2b faisant office de moyen d'introduction dudit élément longiligne 2 (figure 4). L'élément longiligne 2 est introduit ainsi jusqu'à ce que la face de contact 3a de la première pièce d'appui 3 vienne en appui et en butée contre la surface extérieure du pilier 16 (figure 5). Puis le praticien met en tension l'élément longiligne 2 au moyen d'un organe de traction 19 passé par le conduit intérieur d'un poussoir 20 et attaché à son extrémité courbe 2b. La seconde pièce d'appui 8 peut être enfilée facilement à partir de l'extrémité courbe et rigide 2b puis poussée au travers dudit conduit intérieur au moyen d'un piston actionné par le praticien. Le praticien dispose alors la seconde pièce d'appui 8 contre le second pilier 15 en actionnant à la fois l'organe de traction 19 mettant en tension ledit élément longiligne 2 et le piston pour passer en force la seconde pièce 8 sur chaque pièce de blocage (7 à 4) jusqu'à la pièce de blocage 4 prédéterminée par le praticien selon le degré de distension des piliers 15,16 (figure 6). La seconde pièce d'appui 8 est introduite de sorte que sa face de contact 8b vienne en appui et en butée contre la surface extérieure du second pilier 15. Lors du passage en force de chacune des pièces de blocage (7 à 4) à travers la seconde pièce d'appui 8, les ailettes 11 de la pièce tronconique 9 se déforment radialement pour son passage. Dans l'exemple illustré, c'est la quatrième pièce de blocage 4 qui vient en butée arrière contre la seconde pièce d'appui 8. Selon le degré de distension entre les piliers 15,16, le praticien ajuste le rapprochement des deux piliers 15,16 aux moyens des pièces de blocage qu'il force une à une à travers ladite seconde pièce d'appui 8. Sur la figure 6, la seconde pièce 8 a été passée en force par-dessus les quatre pièces de blocage 4,5,6,7. L'espace délimité entre lesdites pièces d'appui 3,8 présente ainsi au moins une dimension D'inférieure à 10 mm apte à recevoir et enserrer les deux piliers 15,16 du diaphragme. Puis le praticien coupe l'élément longiligne 2 au ras de la quatrième pièce de blocage 4 venant en butée contre la seconde pièce d'appui 8 et retire la portion d'élément longiligne 2 coupée ainsi que le poussoir 20 et le lac 18. De préférence, la bande 12 est pré-positionnée sur la pièce tronconique 9 avant l'implantation. Ainsi, à ce stade, la bande élastique 12 empêche lors du passage en force des pièces de blocage 4,5,6,7, occasionnant la déformation des ailettes, que l'élément longiligne 2 ne passe à travers l'un des découpes latérales et ne vienne cisailler les piliers 15,16 et/ou gêner la manipulation du dispositif 1 par le praticien.

Le dispositif 1 ainsi implanté est parfaitement maintenu en tension par la dite pièce de blocage 4, laquelle maintient également les faces de contact 3a,8b contre les piliers fibreux 15,16. Le dispositif 1 ne comporte pas de partie susceptible de cisailler la surface extérieure des piliers 15,16. Les piliers 15,16 sont maintenus rapprochés durablement du fait notamment du développement de la fibrose cicatricielle dans les faces de contact des deux pièces d'appui, développement qui empêche tout glissement éventuel des pièces d'appui par rapport aux structures anatomiques et par conséquent tout effet éventuel de cisaillement desdites structures anatomiques par l'élément longiligne qui les traverse de part en part. Le mode opératoire qui a été présente ci-dessus pour le dispositif 1 doit être légèrement complété pour les variantes de réalisation dans lesquelles la pièce d'appui est composée d'un corps principal et d'une pièce souple qui se projette radialement selon tout ou partie du pourtour dudit corps principal, comme illustré aux figures 14 et 15. Dans ce cas, s'agissant d'une intervention par laparoscopie, le dispositif est introduit dans un trocart dont le diamètre intérieur est légèrement supérieur au diamètre du corps principal, la pièce souple étant en position repliée sur elle-même à l'intérieur dudit trocart. Une fois que la pièce d'appui, que ce soit la première ou la seconde pièce, est mise en place par le praticien comme indiqué ci-dessus, celui-ci réalise le déploiement de la pièce souple pour venir l'appliquer contre la surface extérieure de la structure anatomique correspondante.

Si la pièce souple est une pièce indépendante du corps principal de la pièce d'appui, le praticien doit également faire coulisser le long de l'élément longiligne ladite pièce souple de manière à ce qu'elle vienne en contact d'abord avec la face intérieure de la pièce d'appui et ensuite avec la surface extérieure de la structure anatomique correspondante. Certes, le mode opératoire est un peu plus complexe lorsque la pièce souple est indépendante du corps principal de la pièce d'appui mais cet inconvénient est compensé par l'avantage que présente pour le praticien de pouvoir, avant l'implantation, choisir la dimension voulue pour la pièce souple, soit parmi différentes pièces de dimensions différentes, soit en réalisant lui-même le dimensionnement souhaité en pratiquant une découpe d'une pièce de grande dimension.

Lorsque la pièce souple est indépendante du corps principal de la pièce d'appui, elle ne comporte qu'un trou pour le passage par coulissement de l'élément longiligne 2. Dans ce cas, le développement de la fibrose cicatricielle se propage aussi dans ce trou de passage, ce qui assure également le blocage en position de l'élément longiligne 2 par rapport à la pièce souple 3, élimine définitivement le risque de cisaillement des structures anatomiques par ledit élément longiligne 2.

La face extérieure 32b, 33b de la pièce souple 32,33, à savoir celle qui ne vient pas en contact avec la structure anatomique est, au moins dans son extension radiale au-delà du corps principal 31, apte à empêcher le développement de la fibrose cicatricielle, ceci afin d'éviter qu'il puisse y avoir un développement anarchique de fibrose dans le cas où une structure anatomique autre que celle dont le rapprochement est recherché viendrait en contact avec cette face extérieure 32b,33b. Pour empêcher ce développement de fibrose, la face extérieure 32b,33b peut être revêtue d'une enduction ou d'une imprégnation de silicone.

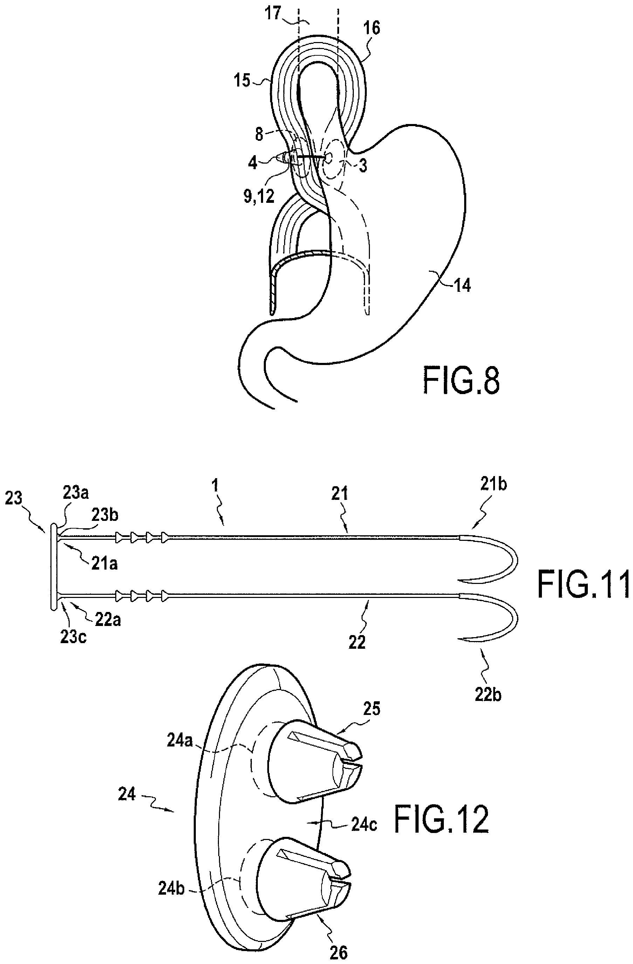

La pièce tronconique 9' représentée aux figures 10 et 11 est une variante de la pièce tronconique 9 décrite ci-dessus. Seuls les éléments de la pièce tronconique 9' qui différent de la pièce 9 sont renumérotés.

La pièce tronconique 9' comporte un épaulement interne 9'a ayant une profondeur h1' dans sa portion d'extrémité 9b formant un logement apte à recevoir une partie de la hauteur h4,h5,h6,h7 de l'une des pièces de blocage 4,5,6,7. La largeur l1' de la bande 12' est de l'ordre de celle de la hauteur h1 de la pièce tronconique 9' majorée de la hauteur h4,h5,h6,h7 moins la profondeur h1' de l'épaulement interne 9a en sorte qu'en fonctionnement (tel que représenté en partie à la figure 10), la bande 12' recouvre à la fois les découpes latérales 10 et la portion de pièce de blocage débouchant de l'épaulement 9a. Ainsi, l'élément longiligne 2 ne risque pas de cisailler les structures anatomiques voisines en passant à travers l'une des découpes, et l'extrémité amincie, voire pointue, de la pièce de blocage ne risque pas de perforer lesdites structures.

De préférence, les corps principaux 30,80 des première 3 et seconde 8 pièces d'appui, y compris les pièces tronconiques 9,9' ainsi que les pièces de blocage 4,5,6,7 sont moulées, de préférence dans un polymère choisi parmi les polymères suivants : PEEK (polyétherétherkétone), POM (polyoxyméthylène), PET (polyéthylènetéréphtalate), PP (polypropylène), PE (polyéthylène).

Le protecteur 12" représenté à la figure 13 est une variante du protecteur ou bande 12' représenté à la figure 10. Seuls les éléments de la pièce tronconique 9' qui différent de la pièce 9 sont renumérotés à la figure 13. Ce protecteur 12" est moulé en même temps que la seconde pièce d'appui 8 et la première pièce tronconique 9', de préférence dans un polymère choisi parmi les polymères suivants : PEEK (polyétherétherkétone), POM (polyoxyméthylène), PET (polyéthylènetéréphtalate), PP (polypropylène), PE (polyéthylène). Le protecteur 12", étant dans ce cas rigide, se présente sous la forme d'un cylindre s'étendant de la face 8c de la seconde pièce d'appui 8. Le protecteur 12" recouvre les découpes latérales 10 de la pièce tronconique creuse 9' sans entrer en contact direct avec ladite pièce tronconique 9' et donc ses ailettes 11 de sorte de ménager un espace libre, délimité entre ladite pièce tronconique 9' et ledit protecteur 12', dans lequel lesdites ailettes 11 peuvent se déformer radialement lors du passage en force de la pièce de blocage 4,5,6,7.

Le dispositif 1' représenté à la figure 11 diffère du dispositif 1 en ce qu'il comprend deux éléments longilignes 21,22. La première pièce d'appui 23 présente une face de contact plane 23a suffisante pour être fixée en deux zones de fixations différentes 23b,23c aux extrémités 21a,22a des premier 21 et second 22 éléments. Chaque élément longiligne 21,22 comporte quatre pièces de blocage, de préférence agencées telles que décrites ci-dessus. La seconde pièce d'appui 24, à la figure 12, comporte un premier 24a et un second 24b orifices de passage pour être montée coulissante à la fois respectivement sur le premier 21 et le second 22 éléments longilignes. A l'opposé de la première pièce d'appui 23, les extrémités libres 21b,22b des éléments 21,22 sont rigides et courbes. La seconde pièce d'appui 24 comporte deux pièces tronconiques 25,26 se projetant de sa face extérieure 24c identiques à la pièce tronconique 9 du dispositif 1 et comportant chacune en fonctionnement une bande du type de la bande 12.

En fonctionnement, le praticien passe successivement le premier élément 21 puis le second élément 22 à travers les piliers du diaphragme jusqu'à ce que la première pièce d'appui 23 vienne en butée contre un premier pilier. Puis il enfile la seconde pièce d'appui 24 sur les premier 21 et second 22 éléments longilignes à l'aide des premier 24a et second 24b orifices de passage. Chacune des pièces de blocage disposées sur la longueur des premier 21 et second 22 éléments est forcée à travers les premier 24a et second 24b orifices de passage. De préférence, afin d'obtenir un espace délimité par une dimension sensiblement constante, correspondant à l'écartement entre les première 23 et seconde 24 pièces d'appui, le praticien bloque la seconde pièce d'appui 24 à l'aide de deux pièces de blocage disposées sensiblement à une distance du même ordre de la première d'appui 23.

Le second élément longiligne 22, tout comme le premier élément longiligne 21, ne cisaille pas les piliers et renforce la pression de serrage exercée par le dispositif 1' et notamment les première 23 et seconde 24 pièces d'appui. Le dispositif 1' permet l'emploi de pièces d'appui 23,24 ayant des faces de contact plus importantes que celles des pièces 3,8 du dispositif 1, et de mieux équilibrer la répartition des tensions exercées par les premier 21 et second 22 éléments longilignes entre lesdites pièces 23,24.

De préférence, les pièces tronconiques 25,26 comportent chacune un protecteur se présentant sous la forme d'un tube dans un matériau élastique adhérent équivalent au protecteur 12 du dispositif 1.

Bien sûr, selon la pathologie, il est possible d'implanter un ou plusieurs dispositifs 1 décrits aux figures 1A-1D à 8 et/ou un ou plusieurs dispositifs 1' décrits aux figures 9A et 9B.

Dans les exemples de réalisation décrits ci-dessus, la première et la seconde pièces d'appui sont constituées d'un corps principal et d'une pièce rapportée qui présentent les propriétés requises sur le développement de la fibrose cicatricielle. Ceci n'est pas limitatif de la présente invention. La première et la seconde pièces d'appui peuvent être d'un seul tenant, la face de contact présentant les propriétés permettant le développement de la fibrose pouvant résulter de la structure elle-même de la pièce d'appui ou d'un traitement approprié, notamment conférant localement à ladite pièce d'appui une porosité adéquate, avec des pores ouverts autorisant le développement de ladite fibrose.

## Revendications

1. Dispositif implantable (1,1') pour le rapprochement de structures anatomiques fragiles, comprenant :
a) un élément longiligne (2,21,22), destiné à traverser de part en part les structures anatomiques (15,16) à rapprocher ;
b) au moins une première pièce d'appui (3,23,30) disposée sur la longueur dudit élément longiligne (2,21,22) et qui comporte une face de contact (3a,23a) destinée à s'appliquer sur l'une des structures anatomiques (15,16) à rapprocher et présentant, au moins en partie, des pores ouverts permettant le développement d'une fibrose cicatricielle ;
c) au moins une pièce de blocage (4,5,6,7) disposée sur la longueur dudit élément longiligne (2,21,22), à une distance donnée D de la face de contact (3a,23a,31a) de ladite première pièce d'appui (3,23,30) ;
d) au moins une seconde pièce d'appui (8,24) qui comporte une face de contact (8b) destinée à s'appliquer sur une autre des structures anatomiques (15,16) à rapprocher et présentant, au moins en partie, des pores ouverts permettant le développement d'une fibrose cicatricielle, ladite seconde pièce d'appui (8,24) étant apte à coulisser sur la longueur de l'élément longiligne (2,21,22) et étant conformée en sorte que, lors de l'implantation, la pièce de blocage (4,5,6,7) est forcée à travers la seconde pièce d'appui (8,24) et vient en butée arrière contre celle-ci,
chaque première pièce d'appui (3,23,30) étant constituée d'un corps principal rigide ou semi-rigide (31), et d'une pièce souple (32,33) qui se projette radialement selon tout ou partie du pourtour du corps principal (31) et qui est apte à passer d'une position d'introduction dans laquelle elle est repliée sur elle-même à une position d'implantation dans laquelle elle est déployée pour venir s'appliquer sur l'une des structures anatomiques à rapprocher, la face de la pièce souple (32,33) qui, lors de l'implantation, est destinée à être appliquée sur la structure anatomique (15,16) étant apte à développer une fibrose cicatricielle.

2. Dispositif selon la revendication 1, **caractérisé en ce que** la seconde pièce d'appui est constituée d'un corps principal (80) et d'une pièce notamment textile (81), qui est plaquée sur la face intérieure du corps principal (80) et qui fait office de face de contact (8b).

3. Dispositif selon l'une quelconque des revendications 1 et 2, **caractérisé en ce que** le corps principal (31) est une pièce de configuration circulaire faisant de l'ordre de 8 mm de diamètre, l'extension radiale (32,33) au-delà du corps principal (31) présentant un diamètre de l'ordre de 20mm.

4. Dispositif selon l'une des revendications 1 à 3, **caractérisé en ce que** la pièce souple (32) a une configuration annulaire et est solidarisée au corps principal (31) selon son bord central (32a).

5. Dispositif selon l'une des revendications 1 à 3, **caractérisé en ce que** la pièce souple (33) a une configuration elliptique ou circulaire et est percée d'un trou (34) de passage de l'élément longiligne (2).

6. Dispositif selon la revendication 5, **caractérisé en ce que** la pièce souple (33) est indépendante du corps principal (31) de la pièce d'appui, étant apte à coulisser par le trou (34) de passage le long de l'élément longiligne (2) lors de l'implantation.

7. Dispositif selon l'une ou l'autre des revendications 1 à 6, **caractérisé en ce que** la pièce formant en tout ou partie la face de contact apte à développer la fibrose cicatricielle est une pièce textile notamment un non-tissé, de préférence un polyéthylènetéréphtalate.

8. Dispositif (1') selon l'une des revendications 1 à 7, **caractérisé en ce qu'**il comprend un second élément longiligne (22) de rapprochement desdites structures anatomiques (15,16), la première pièce d'appui (23) étant fixe ou fixable dans une position donnée dudit second élément (22), et au moins une pièce de blocage fixée sur la longueur dudit second élément à une distance donnée D de ladite première pièce d'appui (23) dans ladite position, la seconde pièce d'appui (24) étant apte à coulisser sur la longueur dudit second élément longiligne (22) en sorte que, lors de l'implantation, ladite pièce de blocage est forcée à travers ladite seconde pièce d'appui (24) et vient en butée contre ladite seconde pièce d'appui (24).

9. Dispositif (1,1') selon l'une des revendications 1 à 8, **caractérisé en ce que** le premier (2,21), et éventuellement le second (22), élément longiligne comporte plusieurs pièces de blocage successives (4,5,6,7), de préférence ayant une hauteur (h4,h5,h6,h7) du même ordre, à des distances prédéterminées D (D1,D2,D3,D4) de ladite première pièce d'appui (3,23), de préférence la distance D (D1,D2,D3, D4) est comprise dans l'intervalle [10 ; 25] mm.

10. Dispositif (1,1') selon l'une des revendications 1 à 9, **caractérisé en ce que** la seconde pièce d'appui (8,24) présente un premier orifice (8a,24a) permettant le passage du premier élément longiligne (2,21), et éventuellement un second orifice (24b) permettant le passage du second élément longiligne (22).

11. Dispositif (1,1') selon la revendication 10, **caractérisé en ce que** la seconde pièce d'appui (8,24) comporte une première pièce tronconique creuse (9,9',25) et éventuellement une seconde pièce tronconique creuse (26), ayant des découpes (10) formant des ailettes latérales (11) et ayant un évidement central (9a) qui prolonge l'orifice de passage (8a,24a,25a), **en ce que** le diamètre de sortie de l'évidement (9a) de ladite pièce tronconique (9,9',25) est inférieur à l'encombrement de la pièce de blocage (4,5,6,7), en sorte que le passage à force de la pièce de blocage (4,5,6,7) à travers la seconde pièce d'appui (8,24) est obtenu grâce à la déformation radiale des ailettes (11).

12. Dispositif (1) selon la revendication 11, **caractérisé en ce que** la première (9'), et éventuellement la seconde, pièce tronconique creuse comporte un épaulement interne (9'a) dans sa portion d'extrémité (9b) formant un logement apte à recevoir tout ou partie de la hauteur (h4,h5,h6,h7) de l'une des pièces de blocage (4,5,6,7).

13. Dispositif (1,1') selon l'une ou l'autre des revendications 11 et 12, **caractérisé en ce que** ladite première (9,9',25) et éventuellement ladite seconde (26), pièce tronconique, est pourvue d'un protecteur (12,12'), de préférence consistant dans une bande dans un matériau élastique, de préférence à base de silicone, ayant une largeur (ℓ1,ℓ1') de l'ordre de la hauteur (h1) de ladite pièce tronconique (9,9',25,26), éventuellement majorée de tout ou partie de la hauteur (h4,h5,h6,h7) de l'une des pièces de blocage (4,5,6,7), et apte à être disposée en fonctionnement sur ladite pièce tronconique (9,9',25,26), et éventuellement ladite pièce de blocage (4,5,6,7), en sorte de recouvrir au moins lesdites découpes latérales (10).

14. Dispositif (1,1') selon l'une des revendications 1 à 13, **caractérisé en ce que** le premier élément longiligne (2,21), et éventuellement le second élément longiligne (22), est un monofilament, une tresse ou un câble rigide de faible diamètre, de préférence de l'ordre de 1,7 mm.

15. Dispositif (1,1') selon l'une des revendications 1 à 14, **caractérisé en ce que** ledit premier (2,21), et éventuellement ledit second (22), élément longiligne présente une extrémité rigide et courbe (2b,21b,22b) permettant son introduction dans lesdites structures anatomiques, notamment à l'opposé de ladite première pièce d'appui (3,23).

16. Dispositif (1,1') selon l'une des revendications 1 à 15, **caractérisé en ce que** la ou les pièces de blocage (4,5,6,7) ont une forme générale tronconique.

17. Dispositif (1,1') selon l'une des revendications 1 à 16, **caractérisé en ce que** le corps principal des première (3,23) et seconde (8,24) pièces d'appui, la ou les pièces de blocage (4,5,6,7), la première pièce tronconique creuse (9,9',25), éventuellement la seconde pièce tronconique creuse (26), le protecteur de la première pièce tronconique creuse (12"), et éventuellement le protecteur de la seconde pièce tronconique creuse, sont moulés, de préférence dans un polymère choisi parmi les polymères suivants : PEEK (polyétherétherkétone), POM (polyoxyméthylène), PET (polyéthylènetéréphtalate), PP (polypropylène), PE (Polyéthylène).

18. Ensemble pour le rapprochement de structures anatomiques fragiles, notamment de structures musculaires telles que la paroi abdominale, les piliers du diaphragme dans le traitement de la hernie hiatale, le coeur, l'estomac ou l'utérus, ou de structures parenchymateuses telles que les reins, le foie et les poumons comportant un dispositif implantable (1,1') selon l'une des revendications 1 à 17 et un kit d'implantation comprenant notamment un organe de traction (19) et un poussoir (20).

## Patentansprüche

1. Implantierbare Vorrichtung (1, 1') zur Zusammenfügung empfindlicher anatomischer Strukturen, umfassend:
a) ein langgliedriges Element (2, 21, 22), das dazu bestimmt ist, die zusammenzufügenden anatomischen Strukturen (15, 16) vollständig zu durchdringen,
b) mindestens ein erstes Auflageteil (3, 23, 30), das über die Länge des langgliedrigen Elements (2, 21, 22) angeordnet ist und das eine Kontaktfläche (3a, 23a) umfasst, die dazu bestimmt ist, auf eine der zusammenzufügenden anatomischen Strukturen (15, 16) aufgelegt zu werden, und zumindest teilweise offene Poren aufweist, welche die Entwicklung einer Narbenfibrose zulassen,
c) mindestens ein Blockierteil (4, 5, 6, 7), das über die Länge des langgliedrigen Elements (2, 21, 22) in einem gegebenen Abstand D von der Kontaktfläche (3a, 23a, 31a) des ersten Auflageteils (3, 23, 30) angeordnet ist,
d) mindestens ein zweites Auflageteil (8, 24), das eine Kontaktfläche (8b) umfasst, die dazu bestimmt ist, auf eine andere der zusammenzufügenden anatomischen Strukturen (15, 16) aufgelegt zu werden, und zumindest teilweise offene Poren aufweist, welche die Entwicklung einer Narbenfibrose zulassen, wobei das zweite Auflageteil (8, 24) geeignet ist, über die Länge des langgliedrigen Elements (2, 21, 22) zu gleiten, und angepasst ist, sodass während der Implantation das Blockierteil (4, 5, 6, 7) über das zweite Auflageteil (8, 24) gezwungen wird und hinten daran aufliegt, wobei jedes erste Auflageteil (3, 23, 30) aus einem starren oder halbstarren Hauptkörper (31) und einem biegsamen Teil (32, 33) gebildet wird, das radial gemäß dem gesamten oder einem Teil des Umfang(s) des Hauptkörpers (31) hervorsteht und das geeignet ist, um von einer Einführposition, in der es um sich selbst gefaltet ist, in eine Implantationsposition überzugehen, in der es sich ausbreitet, um auf eine der zusammenzufügenden anatomischen Strukturen aufgelegt zu werden, wobei die Fläche des biegsamen Teils (32, 33), die während der Implantation dazu bestimmt ist, auf die anatomische Struktur (15, 16) aufgelegt zu werden, dazu geeignet ist, eine Narbenfibrose zu entwickeln.

2. Vorrichtung nach Anspruch 1, **dadurch gekennzeichnet, dass** das zweite Auflageteil aus einem Hauptkörper (80) und einem Teil (81), das im Besonderen aus Textilien besteht, gebildet wird, das auf die Innenfläche des Hauptkörpers (80) gepresst wird und das als Kontaktfläche (8b) dient.

3. Vorrichtung nach einem der Ansprüche 1 und 2, **dadurch gekennzeichnet, dass** der Hauptkörper (31) ein Teil mit kreisförmiger Ausgestaltung in der Größenordnung von 8 mm Durchmesser ist, wobei die radiale Erstreckung (32, 33) über den Hauptkörper (31) hinaus einen Durchmesser in der Größenordnung von 20 mm aufweist.

4. Vorrichtung nach einem der Ansprüche 1 bis 3, **dadurch gekennzeichnet, dass** das biegsame Teil (32) eine ringförmige Ausgestaltung aufweist und mit dem Hauptkörper (31) gemäß seinem zentralen Rand (32a) fest verbunden ist.

5. Vorrichtung nach einem der Ansprüche 1 bis 3, **dadurch gekennzeichnet, dass** das biegsame Teil (33) eine elliptische oder kreisförmige Ausgestaltung aufweist und von einer Durchgangsbohrung (34) des langgliedrigen Elements (2) durchbohrt ist.

6. Vorrichtung nach Anspruch 5, **dadurch gekennzeichnet, dass** das biegsame Teil (33) von dem Hauptkörper (31) des Auflageteils unabhängig ist, wobei es geeignet ist, während der Implantation durch die Durchgangsbohrung (34) entlang des langgliedrigen Elements (2) zu gleiten.

7. Vorrichtung nach einem der Ansprüche 1 bis 6, **dadurch gekennzeichnet, dass** das Teil, welches vollständig oder teilweise die Kontaktfläche bildet, die geeignet ist, die Narbenfibrose zu entwickeln, ein Textilteil, im Besonderen ein Vlies, vorzugsweise ein Polyethylenterephthalat, ist.

8. Vorrichtung (1') nach einem der Ansprüche 1 bis 7, **dadurch gekennzeichnet, dass** sie ein zweites langgliedriges Element (22) zur Zusammenfügung der anatomischen Strukturen (15, 16), wobei das erste Auflageteil (23) in einer gegebenen Position des zweiten Elements (22) fixiert oder fixierbar ist, und mindestens ein Blockierteil umfasst, das über die Länge des zweiten Elements in einem gegebenen Abstand D von dem ersten Auflageteil (23) in der Position fixiert ist, wobei das zweite Auflageteil (24) geeignet ist, über die Länge des zweiten langgliedrigen Elements (22) zu gleiten, sodass während der Implantation das Blockierteil über das zweite Auflageteil (24) gezwungen wird und an dem zweiten Auflageteil (24) aufliegt.

9. Vorrichtung (1, 1') nach einem der Ansprüche 1 bis 8, **dadurch gekennzeichnet, dass** das erste (2, 21) und möglicherweise das zweite langgliedrige Element (22) mehrere aufeinanderfolgende Blockierteile (4 ,5, 6, 7), die vorzugsweise eine Höhe (h4, h5, h6, h7) in der gleichen Größenordnung aufweisen, in vorbestimmten Abständen D (D1, D2, D3, D4) von dem ersten Auflageteil (3, 23) umfassen, vorzugsweise liegt der Abstand D (D1, D2, D3, D4) in dem Intervall [10; 25] mm.

10. Vorrichtung (1, 1') nach einem der Ansprüche 1 bis 9, **dadurch gekennzeichnet, dass** das zweite Auflageteil (8, 24) eine erste Öffnung (8a, 24a), die den Durchgang des ersten langgliedrigen Elements (2, 21) zulässt, und möglicherweise eine zweite Öffnung (24b) aufweist, die den Durchgang des zweiten langgliedrigen Elements (22) zulässt.

11. Vorrichtung (1, 1') nach Anspruch 10, **dadurch gekennzeichnet, dass** das zweite Auflageteil (8, 24) ein erstes hohles, kegelstumpfförmiges Teil (9, 9', 25) und möglicherweise ein zweites hohles, kegelstumpfförmiges Teil (26) umfasst, aufweisend Teilstücke (10), die seitliche Flügel (11) bilden, und aufweisend eine zentrale Aussparung (9a), welche die Durchgangsöffnung (8a, 24a, 25a) verlängert, dadurch, dass der Durchmesser des Ausgangs der Aussparung (9a) des kegelstumpfförmigen Teils (9, 9', 25) kleiner ist als die Blockierung des Blockierteils (4, 5, 6, 7), sodass der Durchgang durch das Blockierteil (4, 5, 6, 7) über das zweite Auflageteil (8, 24) dank der radialen Verformung der Flügel (11) erhalten wird.

12. Vorrichtung (1) nach Anspruch 11, **dadurch gekennzeichnet, dass** das erste (9') und möglicherweise das zweite hohle, kegelstumpfförmige Teil in ihrem Endabschnitt (9b) eine innere Schulter (9'a) umfassen, die eine Aufnahme bildet, die geeignet ist, um die gesamte oder einen Teil der Höhe (h4, h5, h6, h7) von einem der Blockierteile (4, 5, 6, 7) aufzunehmen.

13. Vorrichtung (1, 1') nach einem der Ansprüche 11 und 12, **dadurch gekennzeichnet, dass** das erste (9, 9', 25) und möglicherweise das zweite (26) kegelstumpfförmige Teil (26) mit einem Schutz (12, 12') versehen sind, der vorzugsweise in einem Band in einem elastischen Material, vorzugsweise auf der Grundlage von Silikon, liegt, eine Breite (ℓ1 ,ℓ1') in der Größenordnung der Höhe (h1) des kegelstumpfförmigen Teils (9, 9', 25, 26), möglicherweise zuzüglich der gesamten oder eines Teils der Höhe (h4, h5, h6, h7) von einem der Blockierteile (4, 5, 6, 7), aufweist und geeignet ist, um im Betrieb über dem kegelstumpfförmigen Teil (9, 9', 25, 26) und möglicherweise dem Blockierteil (4, 5, 6, 7) angeordnet zu sein, um zumindest die seitlichen Teilstücke (10) abzudecken.

14. Vorrichtung (1, 1') nach einem der Ansprüche 1 bis 13, **dadurch gekennzeichnet, dass** das erste langgliedrige Element (2, 21) und möglicherweise das zweite langgliedrige Element (22) ein Monofilament, ein Geflecht oder ein starres Kabel mit geringem Durchmesser, vorzugsweise in der Größenordnung von 1,7 mm, sind.

15. Vorrichtung (1, 1') nach einem der Ansprüche 1 bis 14, **dadurch gekennzeichnet, dass** das erste (2, 21) und möglicherweise das zweite langgliedrige Element (22) ein starres und gebogenes Ende (2b, 21b, 22b) aufweisen, das ihre Einführung in die anatomischen Strukturen, im Besonderen gegenüber dem ersten Auflageteil (3, 23), zulässt.

16. Vorrichtung (1, 1') nach einem der Ansprüche 1 bis 15, **dadurch gekennzeichnet, dass** das oder die Blockierteil(e) (4, 5, 6, 7) eine allgemeine kegelstumpfförmige Form aufweist bzw. aufweisen.

17. Vorrichtung (1, 1') nach einem der Ansprüche 1 bis 16, **dadurch gekennzeichnet, dass** der Hauptkörper des ersten (3, 23) und zweiten Auflageteils (8, 24), das oder die Blockierteil(e) (4, 5, 6, 7), das erste hohle, kegelstumpfförmige Teil (9, 9', 25), möglicherweise das zweite hohle, kegelstumpfförmige Teil (26), der Schutz des ersten hohlen, kegelstumpfförmigen Teils (12") und möglicherweise der Schutz des zweiten hohlen, kegelstumpfförmigen Teils vorzugsweise aus einem Polymer geformt sind, das aus den folgenden Polymeren ausgewählt ist: PEEK (Polyetheretherketon), POM (Polyoxymethylen), PET (Polyethylenterephthalat), PP (Polypropylen), PE (Polyethylen).

18. Einheit zur Zusammenfügung empfindlicher anatomischer Strukturen, im Besonderen Muskelstrukturen, wie etwa der Bauchdecke, der Zwerchfellpfeiler bei der Behandlung einer Hiatushernie, des Herzens, des Magens oder der Gebärmutter, oder parenchymatöser Strukturen, wie etwa der Nieren, der Leber und der Lunge, umfassend eine implantierbare Vorrichtung (1, 1') nach einem der Ansprüche 1 bis 17 und ein Implantationskit, umfassend im Besonderen ein Zugglied (19) und eine Druckvorrichtung (20).

## Claims

1. An implantable device (1, 1') for bringing together fragile anatomical structures, comprising:
a) an elongate element (2, 21, 22), intended to cross from side to side the anatomical structures (15, 16) to be brought together;
b) at least one first bearing part (3, 23, 30) disposed over the length of said elongate element (2, 21, 22) and including a contact face (3a, 23a) intended to be applied to one of the anatomical structures (15, 16) to be brought together and having, at least in part, open pores allowing the development of cicatricial fibrosis;
c) at least one blocking part (4, 5, 6, 7) disposed over the length of said elongate element (2, 21, 22) at a given distance D from the contact face (3a, 23a, 31a) of said first bearing part (3, 23, 30);
d) at least one second bearing part (8, 24) including a contact face (8b) intended to be applied to another of the anatomical structures (15, 16) to be brought together and having, at least in part, open pores allowing the development of a cicatricial fibrosis, said second bearing part (8, 24) being able to slide over the length of the elongate element (2, 21, 22) and being shaped so that, during implantation, the blocking part (4, 5, 6, 7) is forced through the second bearing part (8, 24) and abuts thereagainst;
each first bearing part (3, 23, 30) consisting of a rigid or semirigid main body (31) and of a flexible part (32, 33) which projects radially along all or part of the periphery of the main body (31) and which is able to pass from an insertion position in which it is folded over itself to an implantation position in which it is deployed so as to be applied on one of the anatomical structures to be brought together, the face of the flexible part (32, 33) which, during implantation, is intended to be applied on the anatomical structure (15, 16) being able to develop a cicatricial fibrosis.

2. The device according to claim 1, **characterized in that** the second bearing part consists of a main body (80) and of a part in particular textile part (81), which is pressed on the inner face of the main body (80) and which acts as a contact face (8b).

3. The device according to any one of claims 1 and 2, **characterized in that** that the main body (31) is a part with a circular configuration having a diameter of the order of 8 mm, the radial extension (32, 33) beyond the main body (31) having a diameter of the order of 20 mm.

4. The device according to any of claims 1 to 3, **characterized in that** the flexible part (32) has an annular configuration and is secured to the main body (31) according to its central edge (32a).

5. The device according to any of claims 1 to 3, **characterized in that** the flexible part (33) has an elliptical or circular configuration and is pierced with a hole (34) for passage of the elongate element (2).

6. The device according to claim 5, **characterized in that** the flexible part (33) is independent of the main body (31) of the bearing part, being able to slide through the passage hole (34) along the elongate element (2) during implantation.

7. The device according to either of claims 1 to 6, **characterized in that** the part forming, in whole or in part, the contact surface able to develop the cicatricial fibrosis is a textile part, in particular a non-woven fabric, preferably a polyethylene terephthalate.

8. The device (1') according to any of claims 1 to 7, **characterized in that** it comprises a second elongate element (22) for bringing together said anatomical structures (15, 16), the first bearing part (23) being fixed or fixable in a given position of said second element (22), and at least one fixed blocking part over the length of said second element at a given distance D of said first bearing part (23) in said position, the second bearing part (24) being able to slide over the length of said second elongate element (22) so that, during implantation, said blocking part is forced through said second bearing part (24) and abuts against said second bearing part (24).

9. The device (1,1') according to any of claims 1 to 8, **characterized in that** the first (2, 21), and possibly the second (22), elongate element includes a plurality of successive blocking parts (4, 5, 6, 7), preferably having a height (h4, h5, h6, h7) of the same order, at predetermined distances D (D1, D2, D3, D4) of said first bearing part (3, 23), preferably the distance D (D1, D2, D3, D4) is within the range [10; 25] mm.

10. The device (1, 1') according to any of claims 1 to 9, **characterized in that** the second bearing part (8, 24) has a first orifice (8a, 24a) allowing the passage of the first elongate element (2, 21), and possibly a second orifice (24b) allowing the passage of the second elongate element (22).

11. The device (1,1') according to claim 10, **characterized in that** the second bearing part (8, 24) includes a first hollow frustoconical part (9, 9', 25), and possibly a second hollow frustoconical part (26), having cutouts (10) forming lateral fins (11) and having a central recess (9a) which extends the passage orifice (8a, 24a, 25a), **in that** the outlet diameter of the recess (9a) of said frustoconical part (9, 9', 25) is smaller than the overall size of the blocking part (4, 5, 6, 7), so that the forced passage of the blocking part (4, 5, 6, 7) through the second bearing part (8, 24) is obtained thanks to the radial deformation of the fins (11).

12. The device (1) according to claim 11, **characterized in that** the first (9'), and possibly the second, hollow frustoconical part includes an inner shoulder (9'a) in its end portion (9b) forming a housing able to receive all or part of the height (h4, h5, h6, h7) of one of the blocking parts (4, 5, 6, 7).

13. The device (1,1') according to either of claims 11 and 12, **characterized in that** said first (9, 9', 25), and possibly said second (26), part frustoconical, is provided with a protector (12, 12'), preferably consisting of a strip made of an elastic material, preferably silicone-based material, having a width (ℓ1, ℓ1') of the order of the height (h1) of said frustoconical part (9, 9', 25, 26), possibly increased by all or part of the height (h4, h5, h6, h7) of one of the blocking parts (4, 5, 6, 7) and able to be disposed in operation on said frustoconical part (9, 9', 25, 26), and possibly said blocking part (4, 5, 6, 7), so as to cover at least said lateral cut-outs (10).

14. The device (1, 1') according to any of claims 1 to 13, **characterized in that** the first elongate element (2, 21), and possibly the second elongate element (22), is a monofilament, a braid or a rigid cable of small diameter, preferably of the order of 1.7 mm.

15. The device (1,1') according to any of claims 1 to 14, **characterized in that** said first (2, 21), and possibly said second (22), elongate element has a rigid and curved end (2b, 21b, 22b) allowing its insertion in said anatomical structures, in particular opposite said first bearing part (3, 23).

16. The device (1, 1') according to any of claims 1 to 15, **characterized in that** the blocking part(s) (4, 5, 6, 7) have a generally frustoconical shape.

17. The device (1, 1') according to any of claims 1 to 16, **characterized in that** the main body of the first (3, 23) and second (8, 24) bearing parts, the blocking part(s) (4, 5, 6, 7), the first hollow frustoconical part (9, 9', 25), possibly the second hollow frustoconical part (26), the protector of the first hollow frustoconical part (12"), and possibly the protector of the second hollow frustoconical part, are molded, preferably in a polymer selected from the following polymers: PEEK (polyetheretherketone), POM (polyoxymethylene), PET (polyethylene terephthalate), PP (polypropylene), PE (polyethylene).

18. A set for bringing together fragile anatomical structures, in particular muscle structures such as the abdominal wall, the pillars of the diaphragm in the treatment of hiatal hernia, heart, stomach or uterus, or parenchymal structures such as kidneys, liver and lungs including an implantable device (1, 1') according to any of claims 1 to 17 and an implantation kit comprising in particular a traction member (19) and a pusher (20).
